# EUROPEAN PATENT APPLICATION

(11) **EP 2 682 107 A1**
(43) Date of publication of application: **08.01.2014**
(21) Application number: 13174861.8
(22) Date of filing: 03.07.2013
(51) Int. Cl.: A61K 9/14, A61K 9/16, A61K 33/14, A61K 33/30

(54) **Palatable electrolyte-based composition for oral rehydration**

(30) Priority: 04.07.2012 IT MI20121175
(71) Applicant: DMF Dietetic Metabolic Food S.R.L., 20812 Limbiate (IT)
(72) Inventor: Vicentini, Ultimo Vito, 20812 Limbiate (IT)
(74) Representative: Coppo, Alessandro

(57) **Abstract**

The present invention concerns, in one aspect, a palatable, electrolyte-based composition for oral rehydration comprising potassium and chlorine, zinc mineral salts and a xanthan gum-based complex polysaccharide to improve palatability. The composition of the invention can be in granular or powder form for dissolution in a liquid to reconstitute an electrolyte solution for rehydration. The composition of the invention finds application in the treatment of states of dehydration in adults and children resulting from diarrhoeal syndrome in particular.

## Description

### FIELD OF THE INVENTION

The present invention concerns a palatable, electrolyte-based composition for oral rehydration.

The present invention originates in the field of nutritional products or salt supplements and in particular in the field of electrolyte supplements for the treatment of states of dehydration in the human body.

### BACKGROUND OF THE INVENTION

Maintaining the homeostasis, i.e. the volumes and the composition of bodily fluids within the physiological limits is of fundamental importance for the proper functioning of the human body.

Changes in the hydroelectric metabolism and in the acid-base balance in the human body can indeed determine pathological processes of severity proportional to the extent of the water-salt imbalance.

A state of dehydration occurs when the water balance is negative, i.e. the body loses more liquids than it takes in and thus finds itself in a state of deficiency that prevents physiological functions from being carried out.

Generally, a state of dehydration of the body manifests with typical symptoms such as dry mouth, drowsiness or fatigue, thirst, decreased urination, dry skin, headache with possible dizziness.

The causes that can determine the onset of a state of dehydration are extremely varied and comprise a) the reduced administration of fluids, as in the case of certain dietary regimens, digestive system lesions, unavailability of fluids, loss of consciousness, b) the loss of bodily fluids through the skin, as in the case of burns, hyperthermia, excessive sweating, c) the loss of bodily fluids through urine, as in case of diabetes, of adrenal insufficiency, of kidney disease with loss of salts, d) the loss of fluids though the respiratory system as in the case of hyperventilation or of the reduced moisture content of the oxygen breathed in, e) losses inside the body, due to sequestration, such as in the cases of the third abdominal space (ascites) or chest (chylothorax) or of hypoproteinaemias and f) the profuse gastrointestinal loss of fluid, such as in the case of diarrhoea and vomiting.

The states of dehydration of the human body resulting from a diarrhoeal syndrome are one of the most frequent causes of morbidity and mortality with considerable social and economic implications. It is estimated that there are 1.5 billion episodes of diarrhoea globally per year and that the acute diarrhoea is responsible for a greater percentage of about 21% of all deaths in children in babyhood and toddlerhood. Indeed children constitute the category most at risk of suffering from a state of severe bodily dehydration. Individuals in babyhood and toddlerhood are thus particularly vulnerable, as they weigh relatively little and more easily metabolise water and electrolytes. They are also the age group that suffers most frequently of diarrhoeal episodes.

Older people are also subject to risk as with advancing age the body loses the ability to retain water and the sensation of thirst also manifests less frequently while the body has more difficulty adapting to changes in temperature. The elderly also have a tendency to inadequately nourish and hydrate themselves. Moreover, disabilities or inadequate treatment can result in inadequate hydration. These problems are then aggravated, in many cases, by the presence of chronic diseases, such as diabetes, hormonal changes related to the menopause and the administration of certain medicinal products.

Dehydration, particularly in subjects in babyhood and toddlerhood and in the elderly, in most cases requires the establishment of an adequate rehydration therapy aimed at restoring the physiological water-salt balance.

Rehydration therapy provides for introducing into the body adequate volumes of electrolyte-containing fluids through oral or parenteral administration. The latter is however to be reserved for the most severe cases or in situations, such as in diarrhoea and in pernicious vomiting, in which oral administration is not feasible.

In the more common cases of dehydration, it is instead preferred to re-establish normal blood parameters through the oral administration of appropriate electrolyte solutions. This therapeutic practice has proved to be efficient and easy to disseminate even in less developed countries, thanks to the limited cost and the ease of use of the preparations and to the wide availability of salt supplements or of oral rehydrating solutions (ORS).

Albeit highly effective in restoring the water-salt balance of the body when administered over suitable timeframes and in suitable amounts, the electrolyte solutions currently on the market nevertheless have the disadvantage of not being pleasant-tasting, having a bitter and metallic taste on account of the very presence of the electrolytes and trace elements.

This problem, which appears to be more easily overcome in subjects in adulthood, can become a serious obstacle to rehydration when it comes to rehydrating subjects in babyhood and toddlerhood.

One of the aims of the present invention thus consists of improving the organoleptic properties of the electrolyte solutions and of the rehydrating preparations to allow the administration thereof in therapeutically effective quantities even in subjects in babyhood and in toddlerhood and in adults intolerant to the taste of the electrolytes.

Another aim of the invention consists of improving the palatability of rehydration compositions so as to make them more appealing to a broader population of individuals in need of treatment.

### SUMMARY OF THE INVENTION

The inventors of the present invention have identified the possibility of substantially improving the appeal of the electrolyte rehydration solutions by incorporating a specific complex polysaccharide in their formulation.

The improved palatability of the electrolyte solution obtained, by incorporating one specific polysaccharide complex in the rehydration formulation, makes it possible or in any case substantially facilitates the administration of suitable quantities of electrolytes in subjects in need, in particular those in babyhood and toddlerhood.

In view of the objectives as previously set out, the present invention concerns, in a first aspect, a palatable electrolyte-based composition for oral rehydration comprising
i) potassium-, chlorine-based mineral salts, in a therapeutically effective quantity,
ii) at least one zinc-based trace mineral, in a therapeutically effective quantity,
iii) a complex polysaccharide to improve palatability,
said composition being characterized in that the complex polysaccharide for improving palatability is xanthan gum.

In certain embodiments, the composition of the invention is in solid form, typically in granular or powder form for dissolution in a liquid at the time of use to reconstitute an electrolyte-based rehydration solution.

Typically, the mineral salts and the trace minerals present in the palatable composition of the invention dissolve when they come into contact with a liquid to form a solution, the electrolyte flavour of which, is concealed by the presence of xanthan gum, thus increasing its appeal.

According to one embodiment of the invention, the composition of the invention comprises sodium as a further mineral salt.

The rehydration composition of the invention without sodium is specifically indicated for use in the rehydration therapy of adult subjects and the elderly while the formulation that also contains a sodium salt is particularly suitable in the rehydration therapy of subjects in babyhood, toddlerhood or infancy, in general.

In accordance with a second aspect, the present invention provides the use of a complex, xanthan gum-based polysaccharide to improve the palatability of an electrolyte (ORS) or glucose-electrolyte rehydration solution or composition.

In certain embodiments of this second aspect, the present invention provides for the use of xanthan gum in an effective amount for making palatable an electrolyte-based composition or oral rehydration solution (ORS) wherein said composition or ORS comprises
i) potassium-, chlorine-based mineral salts, in a therapeutically effective quantity,
ii) at least one zinc-based trace mineral, in a therapeutically effective quantity.

### DETAILED DESCRIPTION OF THE INVENTION

The applicant has found that the typical metallic and/or bitter taste of an aqueous solution, typically an ORS, containing electrolytes such as for example potassium, chlorine, sodium and certain minerals or trace minerals (also said oligominerals) used in rehydration therapy, is concealed by adding xanthan gum to the solution.

In accordance with a first aspect of the invention, a palatable electrolyte-based for oral rehydration is thus provided, comprising
i) potassium-, a chlorine-based mineral salt, in a therapeutically effective quantity,
ii) at least one zinc-based trace mineral, in a therapeutically effective quantity,
iv) a complex, xanthan-based polysaccharide to improve palatability or taste or flavour.

In a second aspect the invention provides for the use of xanthan gum in an effective amount for making palatable an electrolyte-based composition or oral rehydration solution (ORS) wherein said composition or ORS comprises
i) potassium-, chlorine-based mineral salts, in a therapeutically effective quantity,
ii) at least one zinc-based trace mineral, in a therapeutically effective quantity.

It has surprisingly discovered that the presence of xanthan gum in a composition for oral rehydration or in a ORS substantially improves the organoleptic properties of the electrolyte solution, namely the set of physical and chemical characteristics perceived by the sense organs and, which on the whole provoke a reaction in the to whom it is administered.

In particular, it has been found that incorporating xanthan gum in the composition improves the taste or flavour of an electrolyte solution, typically ORS, containing potassium, chlorine based mineral salts and zinc.
and significantly reduces manifestations of discomfort and repulsion to administration in subjects in babyhood or toddlerhood.

In certain embodiments, the composition of the invention is provided in solid form, typically in granular or powder form for dissolution in a liquid to reconstitute the electrolyte-based rehydration solution.

In the context of the invention, the phrase granular form means that the components of the formulation are in the shape and size of granules of the type conventionally used in the art of formulating products for pharmaceutical, nutritional or dietary use.

By way of example, the granules can vary in shape (spherical, cylindrical, etc.) and in size between 1 and 10 mm in diameter.

The phrase powder form means that the dimensions of the components are smaller than those of the granular form and are also suitable for dissolution in a liquid. For example, in the powder form the particles are reduced to a range in size between 0.0001 mm and 1 mm.

The composition of the invention in solid form, for example granular or powder form, can be dissolved in any physiologically acceptable liquid such as water, tea, chamomile and fruit juices, etc.

Typically, the composition of the invention in solid granular or powder form is dissolved in water to give an electrolyte solution suitable for hydrating dehydrated subjects.

In accordance with certain embodiments, the composition of the invention is provided in the liquid or semi-liquid form, for example as a ready-to-administer gel. Typically, when the rehydration composition of the invention is in the form of a solution, it comprises one or more antimicrobial substances and/or preservatives to prevent any bacterial contamination, the spoilage thereof or organoleptic changes resulting from fermentative bacterial processes.

According to certain embodiments, the palatable composition of the invention contains sodium or a sodium salt as a further electrolyte. The formulations containing sodium/sodium salt as electrolyte are particularly indicated for treating forms of dehydration in subjects in babyhood or toddlerhood. In these subjects of a young age, dehydration, principally resulting from a diarrhoeal syndrome, determines a strong electrolyte imbalance that must be rapidly rebalanced in order to prevent the onset of potentially adverse side effects, of a neurological nature, as well as of cardiocirculatory nature.

It has been verified that in subjects in babyhood and toddlerhood, the administration of the composition containing sodium can take place with limited risks of determining an increase in blood pressure beyond physiological values and thus without substantially increasing the body's level of cardiovascular risk.

The use of xanthan gum as an agent that masks the bitter or metallic taste of the electrolytes and of the mineral salts entails a series of advantages.

Typically, the terms babyhood and infancy have substantially the same meanings in the present application. Typically the composition of the invention can be added to water and used as an oral rehydration solution (ORS) in the management of diarrhoea or colitis in babyhood.

In certain embodiments the palatable electrolyte-based composition of the invention does not contain dietary fiber other than xanthan gum, such as glucomannan or alginate, because the presence of fibers other than xanthan gum in the composition may make difficult the management of diarrhoea and/or gastrointestinal affections with increased motility of intestine.

In certain embodiments of the present application the term oligomineral is to mean a dietary mineral or a trace mineral that is a mineral which is present in traces in water. By way of an example dietary minerals includes iron, cobalt, copper, zinc, molybdenum, iodine, or selenium.

A first advantage consists of the possibility of administering mineral salts or electrolytes in a physiologically active quantity to obtain the rehydration of a body in a sate of dehydration.

A second advantage consists of providing a palatable rehydration composition with a contained cost since xanthan gum is a raw material with a low production and supply cost. This advantage is extremely important in order for allowing a broad dissemination of the palatable composition of the invention at costs that are also acceptable for third world countries where diarrhoea caused by microbiological infections of the water is one of the principal causes of morbidity and mortality.

A further advantage consists of making the mineral salt or electrolyte-based rehydrating solutions appealing, even without any addition of sugars, thus allowing the use thereof even in diabetic subjects.

The xanthan gum used in the present invention is a complex carbohydrate or polysaccharide having a high molecular weight that presents in powder form with high solubility in water but is substantially insoluble in alcohol. The main sugars that constitute it are hexoses such as D-glucose and D-mannose; D-glucuronic and pyruvic acid are also typically present.

The xanthan gum can typically be obtained by means of a bacterial fermentation process of a simple carbohydrate, such as glucose or saccharose for example. Bacterial strains of Xanthomonas campestris are typically used in the microbiological production of xanthan gum. The product obtained by the microbiological fermentation is purified by extraction with ethanol for example, or with propan-2-ol, then dried and ground to produce xanthan gum.

In certain embodiments, the xanthan gum is incorporated in the composition of the invention in a quantity of 2.5 to 50% by weight, preferably of 5 to 20% by weight. The presence of K and Cl salts in the composition of the invention is essential in order to form or reconstitute an aqueous electrolyte solution suitable for reintegrating the states of K⁺ and Cl⁻ ion deficiencies resulting from states of bodily dehydration.

According to certain embodiments of the composition of the invention, the presence of sodium allows the formation of an aqueous electrolyte solution suitable for reintegrating a state of Na⁺ deficiency that typically manifests in cases of infectious diarrhoea.

The presence of an trace mineral, in particular Zn, in the formulation of the invention plays an important role in the cases of dehydration in which the intestinal mucosa is damaged, such as for example, in cases of microbiological infection for example with E. Coli, Vibrio cholerae. It has been verified that the administration of Zn reduces the duration and acute or chronic diarrhoeal symptoms as it reduces the susceptibility of the mucosa to bacterial or viral toxins, assists recovery of the physiological conditions of the intestinal mucosa and improves its absorption of water and electrolytes.

In certain embodiments, the rehydration composition of the invention further contains at least one carbohydrate, typically a sugar, such as glucose, saccharose, dextrose or maltodextrin and mixtures thereof.

According to certain embodiments, the palatable rehydration composition includes a prebiotic, for example a fructoligosaccharides (FOS) such as inulin.

In certain embodiments the palatable rehydration composition of the invention is a palatable oral rehydration solution (ORS). In these embodiments the palatable ORS of the invention contains water.

It was observed that by dissolving in water the palatable composition of the invention in solid form containing a carbohydrate, for example glucose or a fructoligosaccharide, a sugar-electrolyte solution suitable for treating severe states of dehydration principally resulting from diarrhoea caused by Escherichia coli or Rotavirus, is easily and practically reconstituted.

In certain embodiments, the palatable rehydration composition further comprises at least one fructoligosaccharide, which oral administration in the human body promotes the development of a physiological acidophilus flora that competes with the pathogenic flora at the origin of the diarrhoea.

The embodiments of the palatable rehydration composition which in addition to glucose also contain sodium then proved to be particularly suitable for treating severe forms of dehydration resulting from diarrhoea caused by Vibrio cholerae, generally associated with a severe loss of sodium, chlorine and sodium carbonate. In these cases, a typical rehydration treatment provides for the administration of the palatable rehydration composition of the invention, which dissolved in water has an osmolarity of between 300 and 320 mmol/l.

In the composition of the present invention, the mineral salts are present in quantities suitable for restoring physiological values of potassium (K+), chloride (Cl-), and optionally sodium (Na+) electrolytes, in the blood.

In certain embodiments, a quantity of K is present in the invention, which provides an aqueous solution comprising 10-40 mEq/l, preferably between 15 and 25 m Eq/l .

In certain embodiments, a quantity of chloride ions or Cl⁻ that provides an aqueous solution comprising 10-100 mEq/l, preferably 20-60 mEq/l, is present in the composition of the invention.

In certain embodiments, a quantity of Na that provides an aqueous solution comprising 20-100 mEq/l, preferably 50-70 mEq/l, is present in the composition of the invention.

In certain embodiments, Zn is present in the composition of the invention in a quantity of between 1 and 100mg, preferably between 5 and 20mg.

In certain embodiments, a quantity of one sugar, typically glucose which provides an aqueous solution comprising 70-120 mmol/l, preferably 90-110 mmol/I is present in the composition of the invention.

In certain embodiments the composition comprises citrate ions, for example in an amount from 10 mEq/l to 50 mEg/l. Suitable citrate sources are sodium or potassium citrate, citric acid and mixtures thereof.

In certain embodiments, the palatable rehydration composition of the invention contains a quantity of mineral salts or potassium, chlorine, Zn and optionally sodium electrolytes in quantities such as to provide an aqueous solution with osmolarity of from 200 and 350 mOsm/l.

The applicant also observed that adding a green tea extract to a palatable composition of the present invention determines a synergistic treatment effect of the states of dehydration accompanied by vomiting and/or diarrhoea.

In particular, it has been observed that the combination of green tea leaf extract with the palatable rehydration composition of the present invention determines a joint antiemetic-rehydration effect that is particularly appreciable in treating states of dehydration in subjects in babyhood and toddlerhood.

Consequently, according to certain embodiments, the palatable electrolyte-based composition of the invention comprises a green tea extract.

In accordance with another aspect of the present invention, a beverage containing a green tea extract containing a rehydration composition of the type described above, is provided.

The beverage according to this aspect of the invention finds particular application in treating meld and moderate dehydration. The great appeal provided by the presence of the xanthan gum combined with the antiemetic properties of the green tea extract and its great appeal make it particularly indicated for treating and preventing states of dehydration in children, in particular children in babyhood and toddlerhood.

By way of example, the preparation containing the tea extract may be indicated for restoring the water-salt balance in children subject to colitis, even of the recurring type.

In the context of the present invention, the phrase green tea extract means a product conventionally extracted from green tea leaves by means of solvent. In certain embodiments, the extraction is carried out using water as solvent, heated to a temperature above 50 °C, and typically between 70 and 95 °C for a time between 2 minutes and one hour.

In certain embodiments, the green tea extract was prepared by extraction on the dried sheets of green using water at 80 °C for a period of about 15-25, followed by a second extraction with water brought to a temperature between 90 and 97 °C, for a period of time between 5 and 15 minutes.

In accordance with certain embodiments, the composition of the invention further comprises a citrate, for example sodium citrate and/or a bicarbonate, for example sodium bicarbonate.

According to certain embodiments, the composition of the present invention contains one or more vitamins such as, for example, B-group vitamins, vitamin A, C, K, and D.

In certain embodiments, the composition of the invention further contains lutein.

In certain embodiments, the composition of the present invention further contains coenzyme Q10.

Typically, the composition of the invention can contain other substances, such as additives, preservatives, stabilisers, aggregants, thickening agents, dyes and flavourings commonly used in the preparation techniques of pharmaceutical, dietary or nutritional products intended for oral administration.

In certain aspects, the present invention uses a complex, xanthan-gum based carbohydrate as agent to improve the palatability of a rehydration composition. According to another aspect, the present invention provides a palatable hydrating composition of the type previously described for use in treating a form of dehydration resulting from diarrhoea in children, in particular in subjects in babyhood or toddlerhood.

The phrase subjects in babyhood means children up to 2 years of age. The phrase toddlerhood means children between 2 and 5 years of age.

The composition also containing sodium is particularly suitable for these uses. The presence of the xanthan gum in the composition makes it possible or in any case substantially facilitates the administration of suitable quantities of an electrolyte-containing solution.

According to certain embodiments, the form of dehydration resulting from diarrhoea is caused by Rotavirus enteritis. These viral infections have proved to be responsible for more than a third of all the episodes of diarrhoea in children in industrialised, western countries.

According to certain embodiments, the composition of the invention further incorporates a prebiotic and/or a micro-organism suitable for restoring the physiological conditions of the intestinal mucosa, such as Lactobacillus GG, for example.

The present invention will now be described with reference to the following examples, which are provided for illustrative purposes only and are not to be construed as limiting the present invention.

### EXAMPLE 1

Palatable hydrating composition in granular form containing the following ingredients:
maltodextrin, dextrose monohydrate, thickening agent: xanthan gum, sodium chloride, dipotassium phosphate, acidifier: citric acid, inulin (glucose, fructose, saccharose) 90% tit., anti-caking agent: silicon dioxide, cream-vanilla aroma, erythritol, trisodium citrate, green tea leaves (thea sìnensis) maltodextrins), e.g , zinc sulphate monohydrate, vanillin, sweetener, sucralose.

Average analysis of the composition:

| | 'per 1 sachet | Per 100g |
|---|---|---|
| Energy value (kcal/kj) | 14.63 / 61.71 | 276.08 / 1164.44 |
| Protein (Nx6.25) | 0.08g | 1.52g |
| Carbohydrates | 3.11g | 58.61g |
| - of which sugars | 1.45g | 27.40g |
| Fibre | 0.94g | 17.77g |
| Sodium | 0.1g | 1.95g |
| Potassium | 0.08g | 1.48g |
| Chlorine | 0.15g | 2.68g |
| Citrate | 0.2g | 3.62g |
| Zinc | 0.006g | 0.12g |
| Glucose | 0.14g | 2.68g |
| Green tea | 0.02g | 0.38g |
| Osmolarity | 196Mmol/L | |

### EXAMPLE 2

### Palatable hydrating composition in powder form

| Ingredients | mg per sachet |
|---|---|
| | |
| Xanthan gum | 2,901.90 |
| Dextrose monohydrate | 1,448.54 |
| Sodium chloride | 233.76 |
| Sodium citrate | 49.04 |
| Potassium phosphate | 174.16 |
| Citric acid | 154.60 |
| Zinc sulphate | 17.98 |
| Inulin 90% | 111.11 |
| Green tea e.g. tit with 10% caffeine | 20.00 |
| Sweetening excipient flavourings | g.b. |
| Total | 5.3g |

### EXAMPLE 3

Supplement in granular form packaged in sachets of about 5g for the reconstitution of a rehydrating electrolyte solution for oral administration in adult subjects.

| NUTRITIONAL VALUES | | | 100% RDA | | 1 sachet in 250ml of liquid |
|---|---|---|---|---|---|
| Vitamin A | mcg | 800 (100% RDA) | | | 200 |
| Vitamin B1 | mg | 1.4 (100% RDA) | | | 0.35 |
| Vitamin B2 | mg | 1.6 (100% RDA) | | | 0.4 |
| Niacin | mg | 18 (100% RDA) | | | 4.5 |
| Pantothenic acid | mg | 6 (100% RDA) | | | 1.5 |
| Vitamin B6 | mg | 2 (100% RDA) | | | 0.25 |
| Biotin | mcg | 150 (100% RDA) | | | 37.5 |
| Folic acid | mcg | 200 (100% RDA) | | | 50 |
| Vitamin B12 | mcg | 1 (100% RDA) | | | 0.25 |
| Vitamin C | mg | 60 (100% RDA) | | | 15 |
| Vitamin E | mg | 10 (100% RDA) | | | 2.5 |
| Vitamin D | mcg | 5 (100% RDA) | | | 1.25 |
| Vitamin K | mcg | 75 (100% RDA) | | | 18 |
| Lutein | mcg | 1000 | | | 250 |
| Calcium | mg | 800 (100% RDA) | | | 200 |
| Magnesium | mg | 300 (100% RDA) | | | 75 |
| Iron | mg | 14 (100% RDA) | | | 3.5 |
| Phosphorus | mg | 700 (100% RDA) | | | 175 |
| Copper | mg | 1,000 (100% RDA) | | | 250 |
| Chromium | mcg | 40 (100% RDA) | | | 10 |
| Manganese | mg | 2 (100% RDA) | | | 0.5 |
| Potassium | mg | 23.2 | | | 20 |
| Iodine | mcg | 150 (100% RDA) | | | 37.5 |
| Selenium | mcg | 55 (100% RDA) | | | 14 |
| Zinc | mg | 10 (100% RDA) | | | 2.5 |
| Molybdenum | mcg | 50 (100% RDA) | | | 12.5 |
| Chlorine | mg | 21.5 | | | 20 |
| Q10 | mg | 3 | | | 3 |
| Xanthan gum | g | | | | 2.35 |

### EXAMPLE 4

A clinical trial was carried out at the "Federico II" University of Naples, on 80 babies aged between 10 and 180 days in order to check compliance of the composition of Example 2 dissolved in water (ORS).

### METHODS

Type of study: prospective, randomised, multi-centre on outpatients.

### Patients:

Children in babyhood aged between 10 and 180 days with acute gastroenteritis defined as an unequivocal increase in the number of evacuations (> 3/24 h) and/or alterations of the consistency of the faeces.
- Onset of the symptoms: < 48 hours before enrolment
- Evidence of mild-moderate dehydration (loss of 5-10% of body weight) on clinical evaluation: weight loss, sunken anterior fontanelle sunken eyeballs, dry mucous membranes, loss of skin elasticity, respiration rate, arterial pulse, blood pressure, body temperature, diuresis, lacrimation (annexed table 1)

### Exclusion criteria:

- Age > 180gg
- Dystrophy (P/H< 5°pc)
- Duration of the diarrhoea > 48 hours
- Food intolerances/allergies
- Administration of substances that alter the intestinal microflora (probiotics, prebiotics and antibiotics) or that have adsorbent properties (Diosmectite) or that alter
- intestinal secretion (Loperamide)
- Chronic intestinal diseases (coeliac disease, inflammatory bowel disease, cystic fibrosis, other forms of primary pancreatic insufficiency)
- Primary and secondary gastrointestinal malformations resulting from surgical operation (intestinal atresia, short bowel syndrome)
- Primary and secondary immunodeficiencies
- Systemic infections

### Intervention

### Data collection:

Enrolled patients were divided into 2 groups:
Group 1: Conventional ORS (Na+ 60 mmol/l; glucose 75-100mOsmol/l) in sachets for dissolution in water at the time of use,
Group 2: Preparation of Example 2 in sachets to be dissolved at the time of use. Each sachet was dissolved in 100 ml of water.

Enrolled patients were rehydrated by means of the administering of ORS as follows:
mild dehydration: 30-50 ml/kg of ORS in 3-4 hours
moderate dehydration: 50-100 ml/kg of ORS in 3-4 hours

They subsequently received 10 ml/kg/day of ORS to prevent dehydration until termination of the symptoms.

### RESULTS

The difficulties encountered in orally administering adequate quantities of electrolyte glucose solutions (ORS) of the conventional type due to the pronounced salty-metallic taste were overcome in Group 2 with the administration of the formulation of Example 2. Only for Group 2 was it possible to satisfy the first study objective: acceptance of adequate amounts of palatable electrolyte solution (150 ml/kg/day).

In addition, it was found that in Group 2, the amount of palatable electrolyte solution taken was equal to 8 times the conventional amount of electrolyte solution (ORS) taken by the members of Group 1.

## Claims

1. Palatable electrolyte-based composition for use in oral rehydration comprising
i) potassium-, chlorine-based mineral salts, in a therapeutically effective quantity,
ii) at least one zinc-based trace mineral, in a therapeutically effective quantity,
iii) a complex polysaccharide for improving palatability, said composition being **characterized in that** the complex polysaccharide for improving palatability is xanthan gum.

2. Composition according to claim 1, **characterized in that** it comprises an additional sodium-based mineral salt.

3. Composition according to claim 1 or 2, **characterized in that** it further comprises at least one carbohydrate, preferably a sugar selected from glucose, saccharose, dextrose or maltodextrin and mixtures thereof.

4. Composition according to any one of claims 1-3, **characterized in that** it further comprises a green tea extract.

5. Composition according to any one of claims 1-4, **characterized in that** it further comprises at least one vitamin and/or one probiotic and/or one prebiotic.

6. Composition according to any one of claims 1-5, **characterized in that** it is in granular or powder form, soluble in a liquid to reconstitute a palatable electrolyte solution, or in liquid or semi-liquid ready-to-use form.

7. Palatable, electrolyte-based composition according to any one of claims 1-6, for use in preventing and/or treating a form of dehydration.

8. Palatable, electrolyte-based composition for use according to claim 7, wherein the form of dehydration results from diarrhoea, in particular in subjects in babyhood or toddlerhood.

9. Palatable, electrolyte-based composition for use according to claim 8, wherein the diarrhoea is caused by the presence of a pathogenic micro-organism in the intestine, such as Rotavirus, Escherichia coli or Vibrio cholerae.

10. Use of xanthan gum in an effective amount for improving the palatability or taste of an electrolyte-based composition or an oral rehydration solution (ORS) wherein said composition or ORS comprises
i) potassium-, chlorine-based mineral salts, in a therapeutically effective quantity,
ii) at least one zinc-based trace mineral, in a therapeutically effective quantity.

11. Use according to claim 10 wherein the electrolyte-based composition or the oral rehydration solution has a composition according to anyone of claims 1-6.

12. Use according to claim 10 or 11 wherein xanthan gum is present in an amount of 2.5 to 50% by weight, preferably of 5 to 20% by weight.
